(19) Europäisches Patentamt
European Patent Office
Office européen des brevets

(11) **EP 4 501 256 A1**

(12) **EUROPEAN PATENT APPLICATION**
published in accordance with Art. 153(4) EPC

(43) Date of publication:
**05.02.2025 Bulletin 2025/06**

(21) Application number: **23778768.4**

(22) Date of filing: **23.01.2023**

(51) International Patent Classification (IPC):
**A61B 17/32** $^{(2006.01)}$

(52) Cooperative Patent Classification (CPC):
**A61B 17/32**

(86) International application number:
**PCT/JP2023/001918**

(87) International publication number:
**WO 2023/188729 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.03.2022 JP 2022061072**

(71) Applicant: **FUJIFILM Corporation
Tokyo 106-8620 (JP)**

(72) Inventors:
• **TADA, Nobuyuki
Ashigarakami-gun, Kanagawa 258-8577 (JP)**
• **HIGASHI, Kohei
Ashigarakami-gun, Kanagawa 258-8577 (JP)**

(74) Representative: **HGF
HGF Limited
1 City Walk
Leeds LS11 9DX (GB)**

(54) **SURGICAL TREATMENT DEVICE**

(57)     There is provided a surgical treatment apparatus that directly transmits ultrasound waves and heat from self-heating to a soft tissue such as a blood vessel, shortens a time required for sealing, and minimizes damage caused by spread of heat to a biological tissue in the vicinity of the soft tissue. A surgical treatment apparatus (20) comprises a probe (30) that includes a piezoelectric element (39), a power supply circuit for driving the piezoelectric element (39), a power supply wiring line to the piezoelectric element (39), and an impedance match-ing circuit (44), in which the probe (30) has electrode layers (38) for power supply on both surfaces of the piezoelectric element (39), and the impedance matching circuit (44) sets a driving frequency (f) used in power supply to a frequency lower than a resonance frequency (fr) in an impedance characteristic of a treatment start temperature of the probe (30) and sets a reactance component of impedance at the driving frequency (f) to 0 Ω.

FIG. 17

EP 4 501 256 A1

**Description**

**BACKGROUND OF THE INVENTION**

1. Field of the Invention

The present invention relates to a surgical treatment apparatus.

2. Description of the Related Art

In a medical field, it is known that various types of treatment are performed on a subject by using an ultrasound treatment tool that generates ultrasound oscillation. JP2013-528071A describes that an ultrasound transducer is controlled at two frequencies including a resonance frequency, and each frequency generates an ultrasound wave or generates heat. JP2015-43879A describes a surgical treatment apparatus that supplies power of a resonance frequency (first frequency) of a rectangular piezoelectric body alone and/or a resonance frequency (second frequency) of an entire ultrasound probe which is a laminated structure to a piezoelectric element to perform coagulation/incision treatment of a biological tissue through ultrasound oscillation and heat generation. WO2017/038722A describes an ultrasonic surgical system having a control circuit that controls driving power output from an output circuit such that a temperature of a predetermined part of a cartilage is 120°C or more within 2.2 seconds in cutting for heating the predetermined part of the cartilage through ultrasound oscillation and for scraping off the cartilage. As the ultrasound oscillation, a resonance frequency of the piezoelectric element alone or a piezoelectric element unit is used.

**SUMMARY OF THE INVENTION**

In the ultrasound transducer described in JP2013-528071A, heating is performed without damaging a sample, a time for heating from a room temperature to 95°C is 2 minutes or less, a reaching temperature is low, and a time required for a temperature rise is long. In the surgical treatment apparatus described in JP2015-43879A, there is no description on temperature dependence of an actual size and the dielectric constant (resonance frequency) of the piezoelectric element and a temperature rising speed, and the actual temperature rising time is also unknown. In the ultrasonic surgical system described in WO2017/038722A, the cartilage is scraped off by interaction of oscillation and heat. The temperature of the cartilage is set to a specified temperature within a range of 45°C to 220°C and preferably 120°C to 160°C. A heat generating rate of a soft tissue such as a blood vessel caused by the ultrasound oscillation is significantly lower than that of the cartilage, and it is difficult to raise the temperature of the soft tissue such as a blood vessel to the same extent as a cartilage portion.

In an existing treatment tool using ultrasound waves, some of the ultrasound waves are converted into frictional heat to raise a temperature in the vicinity of a treatment portion such as a blood vessel, and thus a time required for the temperature to rise to a desired temperature is long. Further, a rod-like transmission element for transmitting powerful ultrasound oscillation to the treatment portion is used, and thus the size of the treatment tool is large, and there are no bending properties. Therefore, it is difficult to use the treatment tool in endoscopic/laparoscopic surgery. In addition, in a case where a unit that heats and raises a temperature in the vicinity of the treatment portion, such as a blood vessel, is used in combination, there is a risk in which the time required for the temperature to rise to the desired temperature is long, and heat is widely transmitted to a biological tissue other than the treatment portion, causing damage. Therefore, it is required to efficiently cause self-heating with respect to a sealing treatment portion such as a blood vessel in addition to ultrasound oscillation in order to directly transmit heat from the self-heating to the vicinity of the treatment portion, to shorten the time required for the temperature to rise to the desired temperature, and to minimize damage to the vicinity of the treatment portion caused by the spread of heat to the biological tissue.

An object of the present invention is to provide a surgical treatment apparatus that can directly transmit ultrasound waves and heat from self-heating to a soft tissue such as a blood vessel, shorten a time required for sealing, and minimize damage caused by spread of heat to a biological tissue in the vicinity of the soft tissue.

According to an aspect of the present invention, there is provided a surgical treatment apparatus comprising a probe that includes a piezoelectric element, a power supply circuit for driving the piezoelectric element, a power supply wiring line to the piezoelectric element, a flexible tubular sheath, a control mechanism of an amount of power supplied to the piezoelectric element, and an impedance matching circuit, in which the probe has electrode layers for power supply on both surfaces of the piezoelectric element and an opening and closing mechanism for gripping a biological tissue, the flexible tubular sheath includes the power supply wiring line to the piezoelectric element and a part of the opening and closing mechanism of the probe, a driving frequency used in power supply to the piezoelectric element is a frequency lower than a resonance frequency in an impedance characteristic of the probe, and the impedance matching circuit sets the driving frequency used in the power supply to the piezoelectric element to a frequency lower than the resonance frequency in the impedance characteristic of a treatment start temperature of the probe and a frequency at which a reactance component of impedance at the driving frequency is 0 Ω.

It is preferable that in a case where the driving frequency of the probe is denoted by f, the resonance frequency is denoted by fr, an anti-resonance frequency is denoted by fa, a range of the driving frequency in the impedance characteristic of the treatment start temperature of the probe alone satisfies the following expression (1).

$$4fr - 3fa \leq f < fr \qquad (1)$$

It is more preferable that a range of the driving frequency in the impedance characteristic of the treatment start temperature of the probe alone satisfies the following expression (2).

$$2.5fr - 1.5fa \leq f \leq 1.5fr - 0.5fa \qquad (2)$$

It is most preferable that a range of the driving frequency in the impedance characteristic of the treatment start temperature of the probe alone satisfies the following expression (3).

$$f = 2fr - fa \qquad (3)$$

It is preferable that the piezoelectric element consists of a rectangular planar shape and is polarized in a thickness direction.

It is preferable that one surface of the piezoelectric element is fixed to the probe with a backing member sandwiched therebetween and that the other surface of the piezoelectric element is covered with at least one of or both an acoustic matching layer and an insulating layer.

It is preferable that the biological tissue is a soft tissue including a blood vessel.

It is preferable that the driving frequency is a frequency lower than the resonance frequency and is a frequency at which power consumption contributing to generation of ultrasound oscillation at the driving frequency is in a range of at least 25% or more of power consumption contributing to generation of ultrasound oscillation at the resonance frequency.

It is preferable that in the probe, the resonance frequency in an impedance characteristic of a maximum reaching temperature of the piezoelectric element is equal to or lower than the resonance frequency in the impedance characteristic of the treatment start temperature, and a difference between the resonance frequency in the impedance characteristic of the maximum reaching temperature of the piezoelectric element and the resonance frequency in the impedance characteristic of the treatment start temperature is equal to or less than two times a difference between the resonance frequency and an anti-resonance frequency in the impedance characteristic of the treatment start temperature of the piezoelectric element.

With the present invention, ultrasound waves and heat from self-heating can be directly transmitted to a soft tissue such as a blood vessel, shorten a time required for sealing, and minimize damage caused by spread of heat to a biological tissue in the vicinity of the soft tissue.

**BRIEF DESCRIPTION OF THE DRAWINGS**

Fig. 1A is an explanatory view for describing a configuration of an endoscope system, and Fig. 1B is an explanatory view for describing a configuration of a surgical treatment apparatus.

Fig. 2 is an explanatory view of a state where an ultrasound treatment tool is inserted into an endoscope.

Fig. 3 is a perspective view of a grip piece of the ultrasound treatment tool in a closed state (A) and an open state (B).

Fig. 4 is a cross-sectional view of the grip piece of the ultrasound treatment tool in the closed state (A) and the open state (B).

Fig. 5 is a cross-sectional view taken along line VI-VI of (A) of Fig. 4.

Fig. 6 is a block diagram showing an outline of the surgical treatment apparatus.

Fig. 7 is an equivalent circuit diagram of a piezoelectric element.

Fig. 8 is an explanatory view of an impedance characteristic of the piezoelectric element of the present embodiment.

Fig. 9 is an explanatory view of an admittance characteristic of the piezoelectric element of the present embodiment.

Fig. 10 is an explanatory view of power consumption in a case where an alternating current voltage of 10 V is applied to the piezoelectric element.

Fig. 11 is an impedance matching circuit diagram in which power consumption contributing to self-heating in power consumption of a probe according to the present embodiment exceeds power consumption contributing to generation of ultrasound oscillation.

Fig. 12 is an explanatory view showing a reactance change during impedance adjustment.

Fig. 13 is an explanatory view of a breakdown of probe power consumption during impedance adjustment.

Fig. 14 is an explanatory view of a resonance frequency, an anti-resonance frequency, and a driving frequency range of the probe at each temperature.

Fig. 15 is an explanatory view of temperature dependence of the impedance characteristic.

Fig. 16 is an explanatory view of driving frequency dependence of total power consumption of the probe.

Fig. 17 is an explanatory view before and after impedance adjustment of the probe.

Fig. 18 is an explanatory view of a transition of the probe power consumption after impedance adjustment.

**DESCRIPTION OF THE PREFERRED EMBODIMENTS**

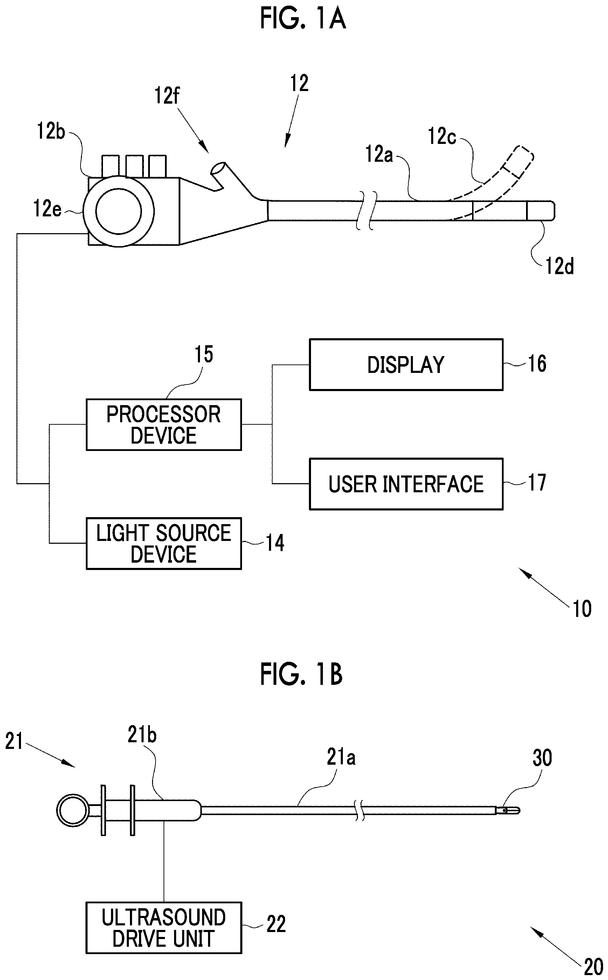

Fig. 1 is composed of Fig. 1A showing an endoscope system 10 used in combination with a surgical treatment apparatus 20 and Fig. 1B showing the surgical treatment apparatus 20 including an ultrasound treatment tool 21. As shown in Fig. 1A, the endoscope system 10 comprises an endoscope 12, a light source device 14, a processor device 15, a display 16, and a user interface (UI) 17. The endoscope 12 images an observation target. The light source device 14 emits illumination light with which the observation target is irradiated. The processor device 15 performs system control of the endoscope system 10. The display 16 is a display unit that displays an observation image or the like based on an endoscope image. The user interface (UI) 17 is an input device that performs setting input or the like to the processor device 15 or the like, such as a mouse and a keyboard.

The endoscope 12 is optically connected to the light source device 14 and is electrically connected to the processor device 15. The endoscope 12 has an insertion part 12a to be inserted into a subject, an operating part 12b provided at a proximal end portion of the insertion part 12a, a bendable part 12c provided on a distal end side of the insertion part 12a, and a distal end part 12d. By operating an angle knob 12e of the operating part 12b, the bendable part 12c is bent. As a result, the distal end part 12d is directed in a desired direction. In addition, a forceps port 12f is provided in the operating part 12b, in addition to the angle knob 12e. The forceps port 12f is an inlet into which an endoscope treatment tool such as the ultrasound treatment tool 21 is inserted. The endoscope treatment tool is used in a state of being inserted into the forceps port 12f.

An observation window and an illumination window are provided in a distal end surface of the distal end part 12d, although not shown. An image sensor (not shown) or the like is disposed behind the observation window, and an optical fiber cable (not shown) is disposed behind the illumination window. Each of the signal line of the image sensor and the optical fiber cable is connected to the processor device 15 and the light source device 14.

The processor device 15 is electrically connected to the display 16 and the user interface 17. The processor device 15 performs image processing or the like on an endoscope image captured by the image sensor and displays the processed image on the display 16.

As shown in Fig. 1B, the surgical treatment apparatus 20 comprises the ultrasound treatment tool 21 that is one of endoscope treatment tools inserted into the subject through the endoscope 12 and an ultrasound drive unit 22 that supplies power. The ultrasound treatment tool 21 has a flexible tubular sheath 21a, an operating part 21b, and a probe 30 and is electrically connected to the ultrasound drive unit 22. The flexible tubular sheath 21a is a tubular sheath formed of a material having flexibility, for example, a soft resin and is inserted into a forceps channel 12g of the endoscope 12. The surgical treatment apparatus 20 having the ultrasound treatment tool 21 is used in, for example, sealing and incision in a case of laparoscopic surgery. The probe 30 is positioned at a distal end of the ultrasound treatment tool 21 and grips a biological tissue. The biological tissue to be gripped is a soft tissue such as a blood vessel.

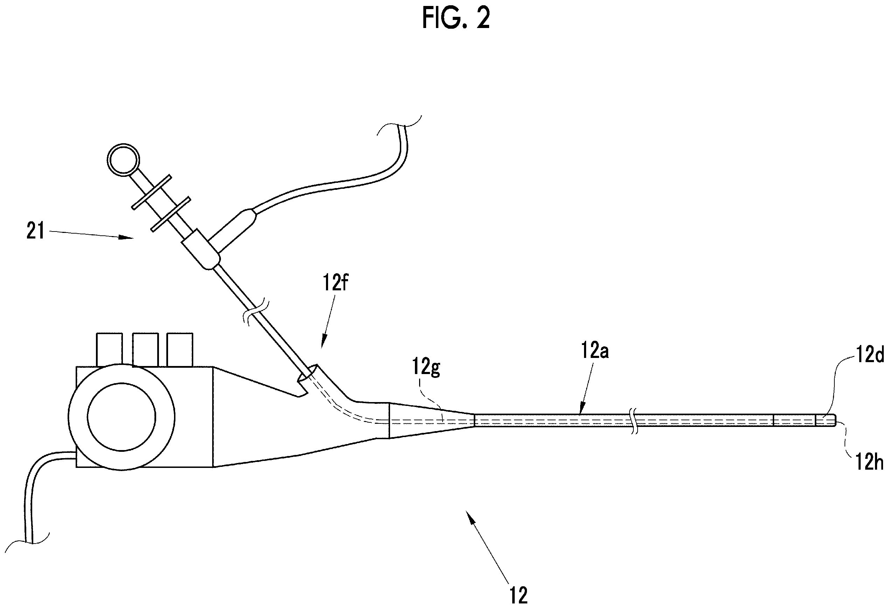

As shown in Fig. 2, the forceps channel 12g for inserting the ultrasound treatment tool 21 is provided in the insertion part 12a having the distal end part 12d. The probe 30 of the ultrasound treatment tool 21 inserted into the forceps port 12f protrudes from a forceps outlet 12h of the distal end part 12d. One end of the forceps channel 12g is connected to a forceps outlet 23, and the other end thereof is connected to the forceps port 12f provided in the operating part 12b. In addition, the forceps channel 12g is also used as a path for feeding a cleaning liquid such as water from the forceps outlet 12h and a path for sucking a body fluid such as blood and contents such as body waste materials. The ultrasound drive unit 22 supplies power to the ultrasound treatment tool 21.

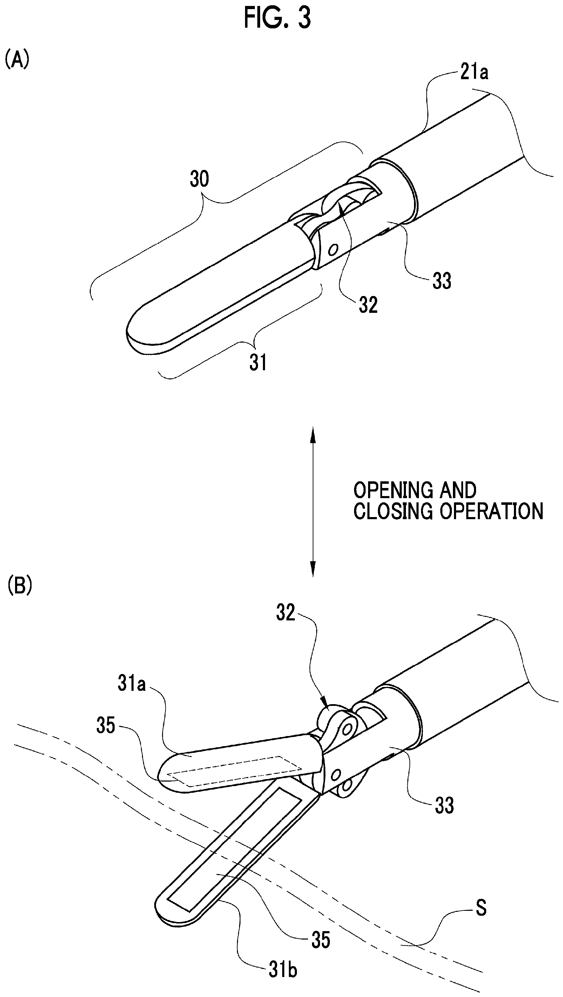

As shown in Fig. 3, the probe 30 has a pair of grip pieces 31 that can be opened and closed in an up-down direction, an opening and closing mechanism 32 that opens and closes the grip pieces 31, and a support member 33 that supports the grip pieces 31. In addition, the flexible tubular sheath 21a partially includes the support member 33. (A) of Fig. 3 shows a state where the grip pieces 31 are closed, and (B) of Fig. 3 shows a state where the grip pieces 31 are open. Each of the grip pieces 31a and 31b is formed in a semi-cylindrical shape, and the probe 30 in the closed state has a cylindrical shape or a shape that is equal to or smaller than at least a circumference of the flexible tubular sheath 21a. As shown in (B) of Fig. 3, acoustic matching layers 35 are provided on inner surfaces of the pair of grip pieces 31 and surfaces facing each other. The opening and closing of the grip pieces 31 is opening and closing of the probe 30.

A structure S such as a blood vessel is passed between the grip piece 31a and the grip piece 31b in a state where the probe 30 is open, and the probe 30 is closed. Accordingly, the inner surfaces of the pair of grip pieces 31a and 31b can be brought close to each other, and the structure S such as a blood vessel, which is a soft tissue, can be gripped with the structure S sandwiched between the pair of grip pieces 31. The structure S such as a blood vessel to be treated, for example, to be sealed is sandwiched between the grip pieces 31 in such a manner. The blood vessel and the like contain a large amount of moisture, which is a factor for delaying a temperature rise. Therefore, by appropriately gripping (pressurizing) the structure S, the moisture contained in the biological tissue gripped by the grip pieces 31 is extruded, and the remaining biological tissue is promoted to be denatured and adhered, thereby enhancing a sealing effect. In the present treatment tool, an appropriate grip pressure at which the sealing effect can be obtained is 10 to 50 N.

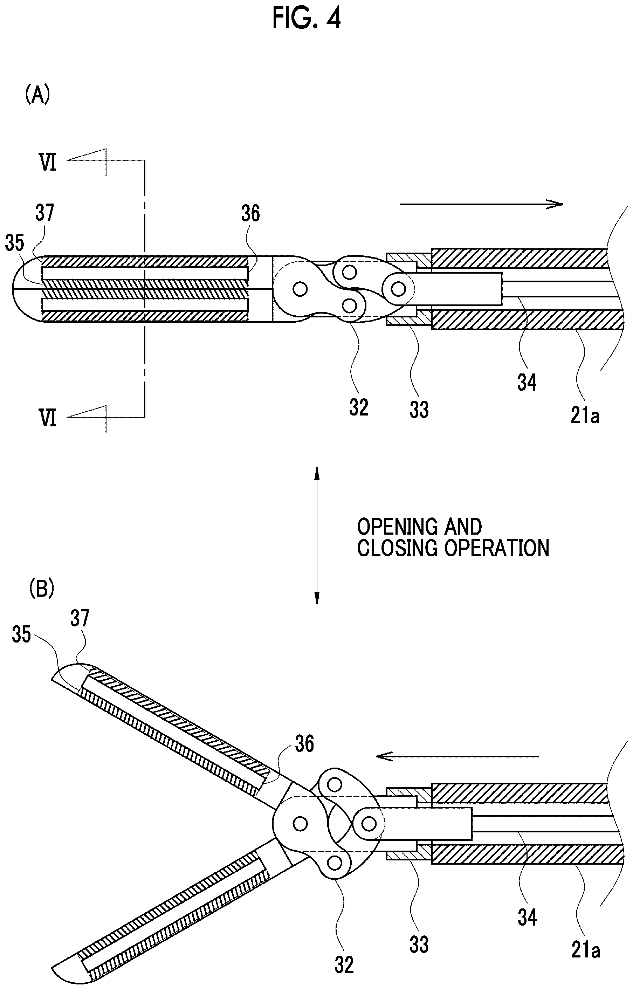

Fig. 4 is a cross-sectional view near the probe 30, and (A) of Fig. 4 shows a cross-sectional view in a state where the probe 30 is closed and (B) of Fig. 4 shows a cross-sectional view in a state where the probe 30 is open. An operation wire 34 is inserted into the flexible tubular sheath 21a. The operation wire 34 transmits an opening and closing operation of the operating part 21b of the ultrasound treatment tool 21 by a user to the opening and closing mechanism 32. In the opening and closing operation, the opening and closing mechanism 32 is brought into a closed state as the operation wire 34 is pulled by the flexible tubular sheath 21a and is brought into an open state as the operation wire 34 is pushed into the distal end part.

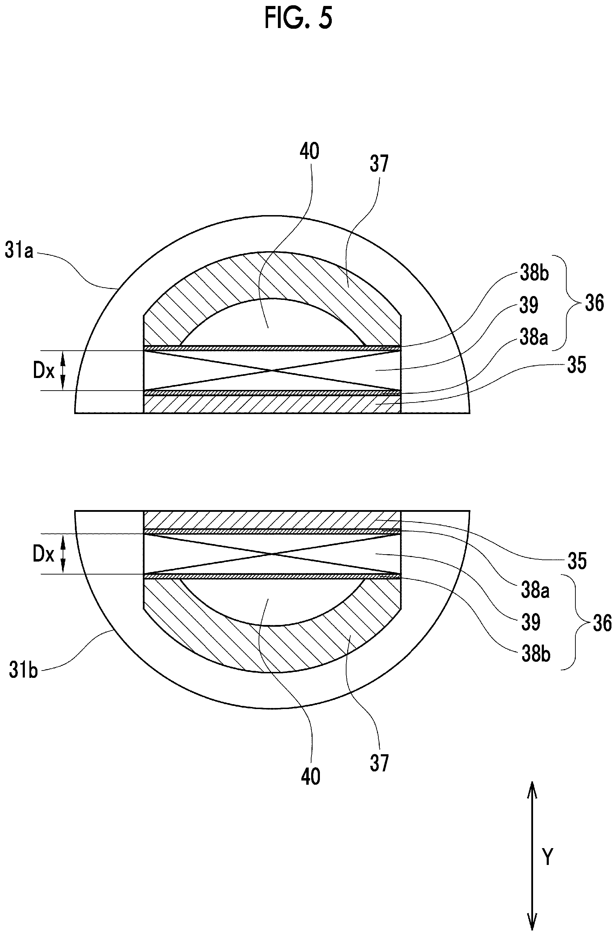

The acoustic matching layers 35, ultrasound oscillators 36, and backing members 37 are embedded in the inner surfaces of the grip piece 31a and the grip piece 31b between which the structure S is sandwiched. By using ultrasound oscillation caused by the ultrasound oscillator 36, the temperature of the structure S such as a blood vessel is raised, and the structure S is sealed. One surface of the ultrasound oscillator 36 is fixed to a probe housing with the backing member 37 sandwiched therebetween, and the other surface is covered with an insulating layer and/or the acoustic matching layer 35. A cross-sectional view taken along line VI-VI in (A) of Fig. 4 will be described with reference to Fig. 5.

As shown in Fig. 5, the acoustic matching layer 35 and the ultrasound oscillator 36 are laminated in parallel inside each of the grip pieces 31a and 31b of the probe 30. The backing member 37 is composed of a material having stiffness, such as hard rubber, or the like and supports the ultrasound oscillator 36 from a grip piece side, which is a back side (an opposite side to the acoustic matching layer 35). It is preferable that the backing member 37 has an arc shape along the semi-cylindrical shape of each of the grip pieces 31a and 31b. The ultrasound oscillator 36 has a configuration where an electrode layer 38a and an electrode layer 38b formed in a plate shape thinner than a piezoelectric element 39 are laminated on both surfaces of the piezoelectric element 39 formed in a plate shape. The piezoelectric element 39 has a rectangular planar shape and is polarized in a thickness direction. Accordingly, a direction of ultrasound oscillation caused by the ultrasound oscillator 36 and a grip direction Y in which the probe 30 grips are parallel to each other. In addition, a direction Dk in which the ultrasound oscillator 36 oscillates is parallel to a thickness direction Dx of the piezoelectric element 39.

The acoustic matching layer 35 is provided in order to match acoustic impedance between a human body of a patient and the ultrasound oscillator 36. The acoustic matching layer 35 is laminated on an outside of the ultrasound oscillator 36, in a strict sense, on a side facing the structure S gripped by the probe 30 with respect to the ultrasound oscillator 36. The acoustic matching layer 35 can increase transmittance of ultrasound waves.

As a material for the acoustic matching layer 35, various organic materials of which acoustic impedance having a value close to the value of the human body, compared to the piezoelectric element 39, can be used. Specific examples thereof include an epoxy resin, silicon rubber, polyimide, and polyethylene. In addition, the acoustic matching layer 35 is formed of a plurality of layers, and a material and the number of layers to be composed are selected as appropriate according to the value of required acoustic impedance.

An air gap layer 40, which is a gap capable of reflecting ultrasound waves with internal air, is formed between the electrode layer 38b on an outer surface side of the plate-shaped ultrasound oscillator 36 and the arc-shaped backing member 37. The air gap layer 40 has a function of reflecting the ultrasound waves emitted from a back side of the ultrasound oscillator 36 and can efficiently transmit ultrasound oscillation to the structure S such as a blood vessel. A material that reflects ultrasound waves may fill instead of the air gap layer 40.

It is preferable to use the piezoelectric element 39 having a mechanical quality coefficient Qm of 500 or more. The mechanical quality coefficient Qm is a coefficient representing an elasticity loss caused by oscillation and is represented by a reciprocal of a mechanical loss coefficient. In a case where the piezoelectric element 39 elastically oscillates, a loss occurs internally and is converted into heat.

In a case where a temperature rise caused by heat generated in the piezoelectric element 39 exceeds 100°C, a possibility of depolarization of the piezoelectric element 39 is also considered. The Curie temperature of the piezoelectric element 39 is desirably equal to or more than approximately 1.5 times and preferably 2 times a target reaching temperature of a surface of the piezoelectric element during power supply. For example, in a case where the target reaching temperature of the surface of the piezoelectric element is set to 200°C, the Curie temperature of the piezoelectric element 39 to be used is set to 300°C or more and preferably 400°C or more. Since there is a possibility in which protein is carbonized at a temperature higher than 200°C, the target reaching temperature is preferably 200°C at its maximum.

The probe 30 including the piezoelectric element 39 has a size that can be used in endoscopic surgery or laparoscopic surgery, and it is desirable that the thickness of the piezoelectric element 39 alone, which is a thickness that can be accommodated in the probe 30, is in a range of 0.2 mm to 1 mm, the width is in a range of 2 mm to 4 mm, and the length is in a range of 10 mm to 30 mm. The width and the length of the piezoelectric element 39 are allowed to be divided into two parts. For example, two parts having a width of 1 mm are arranged to have a total width of 2 mm. In a case where the thickness of the piezoelectric element 39 is thick, heat generated on a surface side of the piezoelectric element 39 not being in contact with a biological tissue is unlikely to be transmitted compared to a surface side in contact with the biological tissue, and a temperature difference between both surfaces tends to be large. On the other hand, in a case where the thickness of the piezoelectric element 39 is thin, the amount of heat generated by the entire piezoelectric element is reduced. In addition, the resonance frequency and the anti-resonance frequency of the piezoelectric element 39 in a state of being accommodated in the probe 30 depend on the thickness of the piezoelectric element, and the frequency tends to be higher as the piezoelectric element 39 is thinner.

Further, in the piezoelectric element 39, a member that holds one surface of the piezoelectric element 39 is held by the probe housing via the backing member 37 having a low thermal conductivity, and a surface on the side in contact with a biological tissue is covered with the acoustic matching layer 35 consisting of a member having a high thermal conductivity or a surface protective layer having a high thermal conductivity. Herein, it is also effective to provide the air gap layer in a part between the ultrasound oscillator 36 and the backing member 37 to more effectively reduce heat transfer properties.

In addition, a difference in thermal expansion factor between the piezoelectric element 39 and the acoustic matching layer 35 causes internal stress between the ultrasound oscillator 36 and the acoustic matching layer 35 during a temperature rise, and thus is a factor for a temperature-dependent fluctuation of a resonance frequency fr and an anti-resonance frequency fa of the piezoelectric element to be described later. Therefore, the acoustic matching layer 35 is selected in consideration of the thermal expansion factor of the piezoelectric element 39.

It is preferable that, even in a case where a temperature reached by the piezoelectric element is high, the probe has a small temperature change in the resonance frequency and the anti-resonance frequency. Specifically, as a condition for selecting the thermal expansion factor of the acoustic matching layer 35, it is preferable that the following two expressions (4) and (5) are satisfied with a resonance frequency frs of the treatment start temperature, a resonance frequency fre of a maximum reaching temperature, and an anti-resonance frequency fas of the treatment start temperature.

Resonance frequency fre of maximum reaching temperature ≤ resonance frequency frs of treatment start temperature                  (4)

(Resonance frequency frs of treatment start temperature - resonance frequency fre of maximum reaching temperature) ≤ (anti-resonance frequency fas of treatment start temperature - resonance frequency frs of treatment start temperature)*2                  (5)

The electrode layers 38a and 38b are connected to the ultrasound drive unit 22 via a signal cable (not shown) constituting a power supply circuit. The signal cable is provided in the flexible tubular sheath 21a. For example, the signal cable is wired along an inner peripheral surface or an outer peripheral surface of the flexible tubular sheath 21a. In a case where the ultrasound treatment tool 21 is connected to the ultrasound drive unit 22, the electrode layers 38a and 38b are electrically connected to the ultrasound drive unit 22 via the signal cable. One of the electrode layers 38a and 38b is connected to the ground via the signal cable or the like, and the other is supplied with power of an alternating current voltage signal to be described later from the ultrasound drive unit 22.

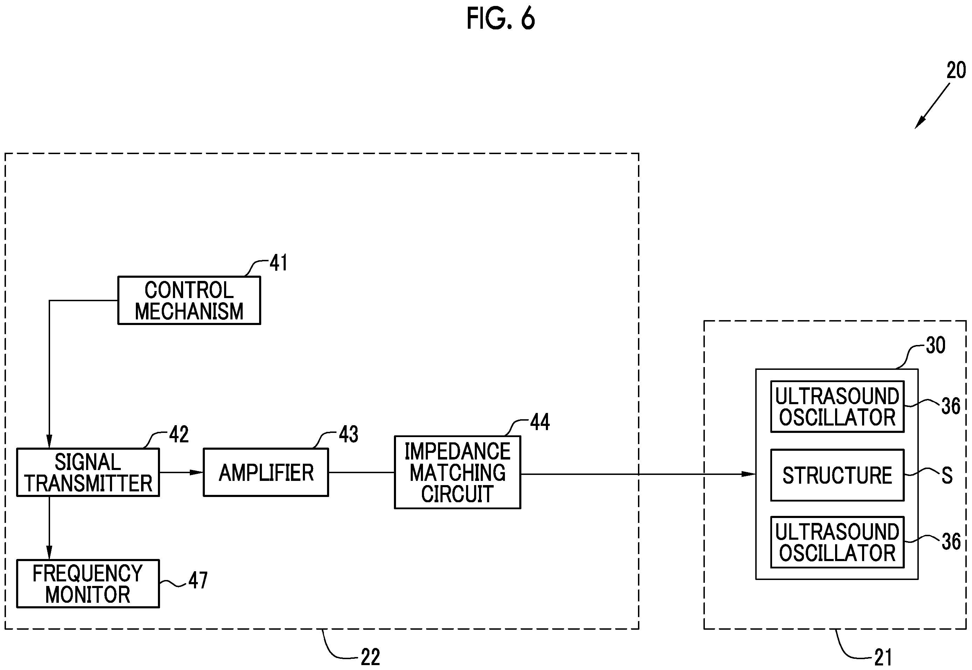

As shown in Fig. 6, the ultrasound drive unit 22 constituting the surgical treatment apparatus 20 comprises a control mechanism 41, a signal transmitter 42, an amplifier 43, an impedance matching circuit 44, and a frequency monitor 47, and the impedance matching circuit 44 is connected to the ultrasound oscillator 36 including the piezoelectric element 39 of the probe 30 of the ultrasound treatment tool 21. The control mechanism 41 controls the amount of power supplied to the piezoelectric element 39, and a path connecting the signal transmitter 42, the amplifier 43, the impedance matching circuit 44, and the ultrasound oscillator 36 includes a power supply circuit for driving the piezoelectric element.

A program related to various types of processing is stored in a program memory (not shown) of the ultrasound drive unit 22. The control mechanism 41 composed of a processor realizes functions of the signal transmitter 42, the amplifier 43, and the impedance matching circuit 44 by executing the program in the program memory.

A frequency actually used in a case of driving the ultrasound oscillator 36 is displayed on the frequency monitor 47. The signal transmitter 42 has a function of generating an alternating current voltage signal having any frequency and waveform and has, for example, the same configuration and function as a known function generator. It is preferable that the ultrasound drive unit 22 is provided with a current probe (not shown) and a current value monitor (not shown) composed of an oscilloscope or the like.

The signal transmitter 42 outputs an alternating current voltage signal having a predetermined frequency. The predetermined frequency is a frequency at which ultrasound oscillation of the piezoelectric element 39 is maximized, that is, the resonance frequency fr. The piezoelectric element 39 has a resonance frequency according to a thickness dimension. Specifically, in a case where the thickness dimension is denoted by D1 (m), a resonance frequency according to the thickness dimension D1 is denoted by fr (MHz), and a piezoelectric sound speed (the speed of a sound wave transmitted in the piezoelectric element 39) is denoted by v (m/sec), a relationship of D1 = v/2fr is established.

The signal transmitter 42 outputs an alternating current voltage signal having a driving frequency f, for example, the resonance frequency fr to the amplifier 43. The amplifier 43 amplifies the alternating current voltage signal output from the signal transmitter 42 to a voltage level at which the ultrasound oscillator 36 can be driven. The impedance matching circuit 44 is connected in series with the amplifier 43 and can match input impedance of the alternating current voltage signal output from the amplifier 43 with impedance of the ultrasound oscillator 36.

The current probe measures a current value input from the impedance matching circuit 44 to the ultrasound oscillator 36 and inputs the current value to the control mechanism 41. In addition, the current value is displayed on the current value monitor. The control mechanism 41 controls the signal transmitter 42 such that the current value measured by the current probe drives the ultrasound oscillator 36. The control mechanism 41 controls the signal transmitter 42 such that an alternating current voltage signal for driving the ultrasound oscillator 36 is continuously supplied. The term "continuously supplied" herein means that the alternating current voltage signal is continuously output from the signal transmitter 42 without interruption at least during the driving of the ultrasound oscillator 36. By driving the ultrasound oscillator 36, treatment by the ultrasound treatment tool 21 can be performed.

The present invention is to make power consumption contributing to self-heating in the piezoelectric element 39 larger than power consumption contributing to ultrasound oscillation and is realized by impedance adjustment. That is, the surgical treatment apparatus 20 having the impedance matching circuit 44 that takes self-heating of the piezoelectric element 39 into consideration is used.

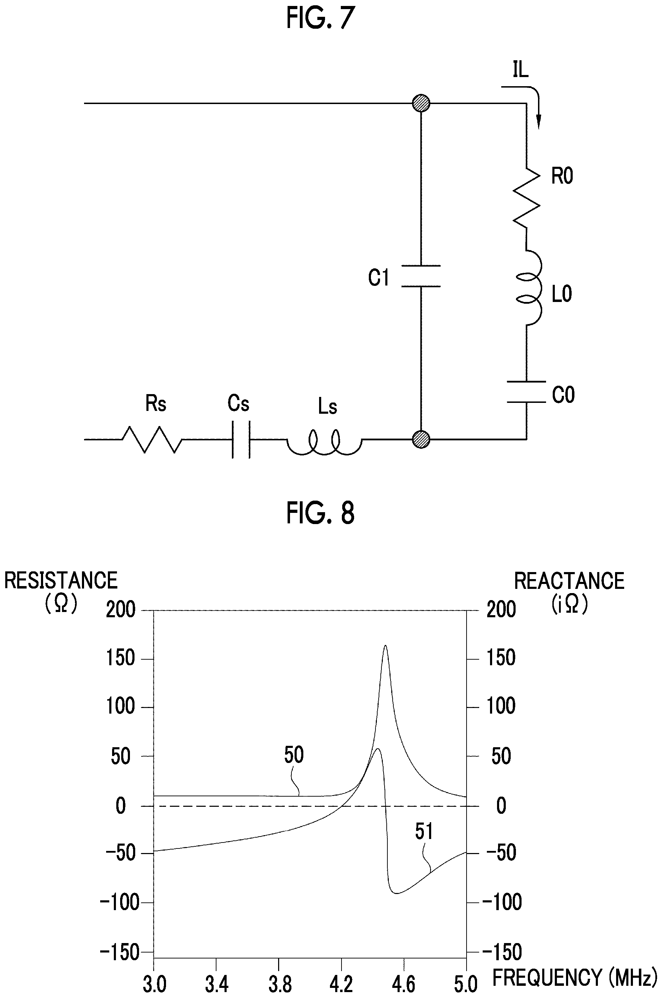

The impedance matching circuit 44 that adjusts impedance is realized based on results obtained in impedance measurement that is an example shown in Figs. 7 to 10. As the example, a temperature rise experiment, in which C-213 (manufactured by Fuji Ceramics Co., Ltd.) having a thickness of 0.5 mm, a length of 3 mm, and a width of 18 mm is adopted as the piezoelectric element 39 and impedance characteristics are measured and adjusted at 25°C, will be described.

Regarding the resonance frequency fr and the anti-resonance frequency fa in the piezoelectric element 39 (the probe 30 having the piezoelectric element 39), a frequency at which a resistance component is maximized (a reactance component is zero) is the anti-resonance frequency fa, and a frequency at which admittance is maximized is the resonance frequency fr.

Fig. 7 is an equivalent circuit of the piezoelectric element 39, and a value of each element can be estimated by measuring impedance characteristics and admittance characteristics. By using this equivalent circuit, it is possible to estimate power consumption contributing to generation of ultrasound oscillation of the piezoelectric element 39 from an electric resistance R0 and power consumption contributing to self-heating from an electric resistance Rs.

Fig. 8 is an impedance characteristic that is a ratio of a voltage to a current in the piezoelectric element 39 according to the present embodiment before impedance adjustment. A curve 50 indicates a resistance component of the impedance characteristic, and a curve 51 indicates a reactance component of the impedance characteristic.

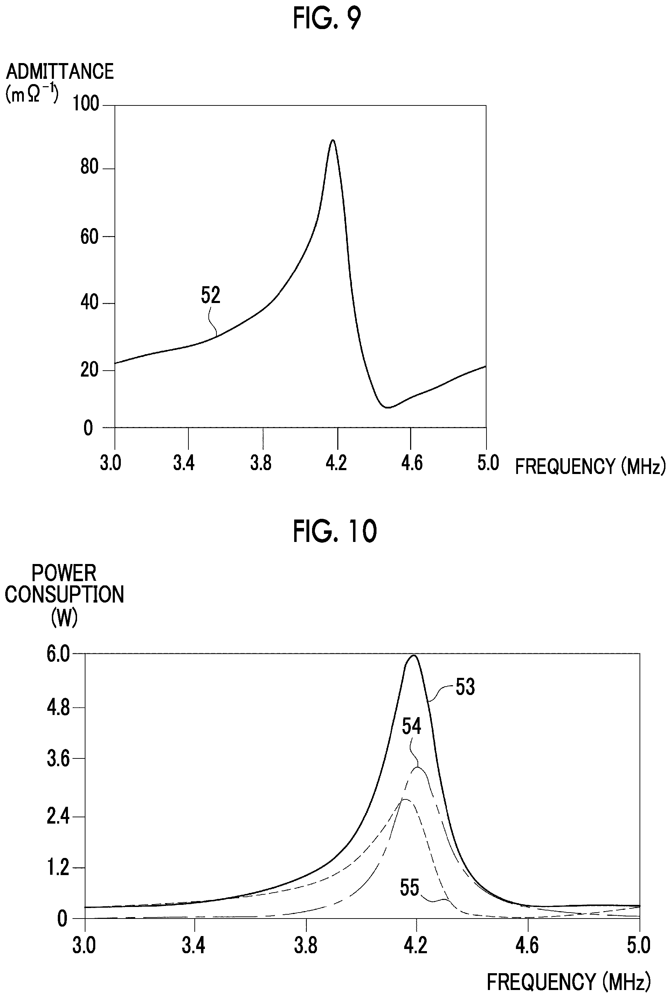

A curve 52 in Fig. 9 is a reciprocal of an admittance characteristic, that is, the impedance characteristic in a case of being measured under the same conditions as in Fig. 8 and indicates a ratio of a current to a voltage. From the impedance characteristic and a reactance characteristic of Fig. 8 and Fig. 9, before impedance adjustment, the resonance frequency fr in a piezoelectric element 38 is 4.2 MHz, and the anti-resonance frequency fa is 4.5 MHz. In general, the driving frequency f is set to a frequency at which ultrasound oscillation in the ultrasound oscillator 36 is maximized, that is, the resonance frequency fr.

Fig. 10 is a breakdown of power consumption in a case where an alternating current voltage of 10 V is applied to the equivalent circuit of Fig. 7. A power consumption curve 53 indicates total power consumption corresponding to a frequency, a power consumption curve 54 indicates power consumption contributing to generation of ultrasound oscillation, and a power consumption curve 55 indicates power consumption contributing to self-heating. The piezoelectric element 39 is driven at a frequency at which ultrasound oscillation is maximized, that is, the resonance frequency fr, and the power consumption contributing to ultrasound oscillation is larger than the power consumption contributing to self-heating. In addition, in order to further reduce self-heating, the resonance frequency fr is set slightly closer to the anti-resonance frequency fa, and a proportion of the power consumption contributing to generation of ultrasound oscillation in the total power consumption increases in some cases. The power consumption contributing to self-heating of the probe 30 is a difference between the total power consumption and the power consumption contributing to generation of ultrasound oscillation.

On the other hand, in the present embodiment, the proportion of power consumption contributing to self-heating in the total power consumption of the piezoelectric element 39 is increased, and based on the above measurement result, the impedance matching circuit 44 is constructed in which a reactance component of impedance is set to 0 Ω at the driving frequency f at which power consumption contributing to generation of ultrasound oscillation and power consumption contributing to self-heating are at a desired ratio. In a case where the driving frequency f is excessively low, ultrasound oscillation is hardly generated. Therefore, the driving frequency f is determined in a range where power consumption contributing to generation of ultrasound oscillation at the driving frequency f is at least 25% or more of power consumption contributing to generation of ultrasound oscillation at the resonance frequency fr.

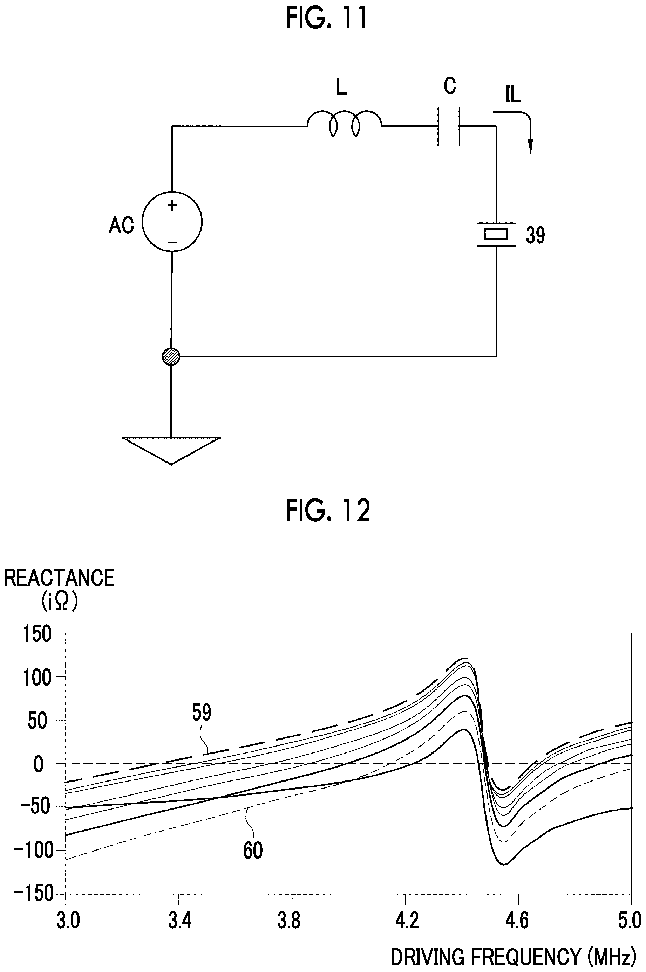

As shown in Fig. 11, a circuit in which impedance adjustment is performed in consideration of self-heating is constructed based on the above measurement result. Specifically, an inductor L, a capacitor C, a power source AC, and the piezoelectric element 39 are included. Impedance is adjusted with respect to the equivalent circuit, and a time required for raising the temperature of a biological tissue is shortened. In a case where the temperature rising time is long, heat transmitted to a treatment portion is diffused in the biological tissue, the temperature of a living body portion in the vicinity of treatment rises, and the living body portion is damaged by heat. Therefore, it is advantageous that the temperature rising time is shorter in order to minimize heat diffusion in the biological tissue.

In electrical characteristics of the probe 30 in a state of gripping an ancillary structure of the probe housing, such as the acoustic matching layer 35 adjusted for impedance and the structure S such as a blood vessel, a frequency at which an absolute value of admittance, which is a ratio of a current to a voltage in an alternating current circuit, is maximized is substituted for the resonance frequency fr, and a frequency at which the absolute value of the admittance is minimized is substituted for the anti-resonance frequency fa.

In general, the impedance matching circuit 44, in which the reactance component of the impedance characteristic of the probe 30 at the resonance frequency fr is set to 0 $\Omega$, optimizes ultrasound oscillation efficiency of the probe at the resonance frequency fr. On the other hand, the present inventors have found that a probe performance required in a case of treatment such as sealing and incision is important in terms of thermal energy as well as ultrasound energy. Therefore, it is desired that the probe 30 is driven under a condition in consideration of an output of thermal energy, that is, the self-heating of the piezoelectric element 39, in addition to the ultrasound energy, and a driving method for this purpose is found.

Fig. 12 shows a change in a reactance accompanied by the impedance adjustment realized in Fig. 11. The capacitor C is adjusted in a range of 0.4 nF to 1.2 nF, and in any case, the inductor L is 4 $\mu$H. A curve 59 indicates a reactance corresponding to the driving frequency f at which the capacitor C is 1.2 nF, and a curve 60 indicates a reactance corresponding to the driving frequency f at which the capacitor C is 0.4 nF. The optimal driving frequency f is low with an increase in the reactance component, and the total power consumption tends to increase, but ultrasound oscillation reduces. The optimal driving frequency f is a frequency at which a reactance component on a low range side of a frequency at which the reactance component is a maximum value is 0 $\Omega$.

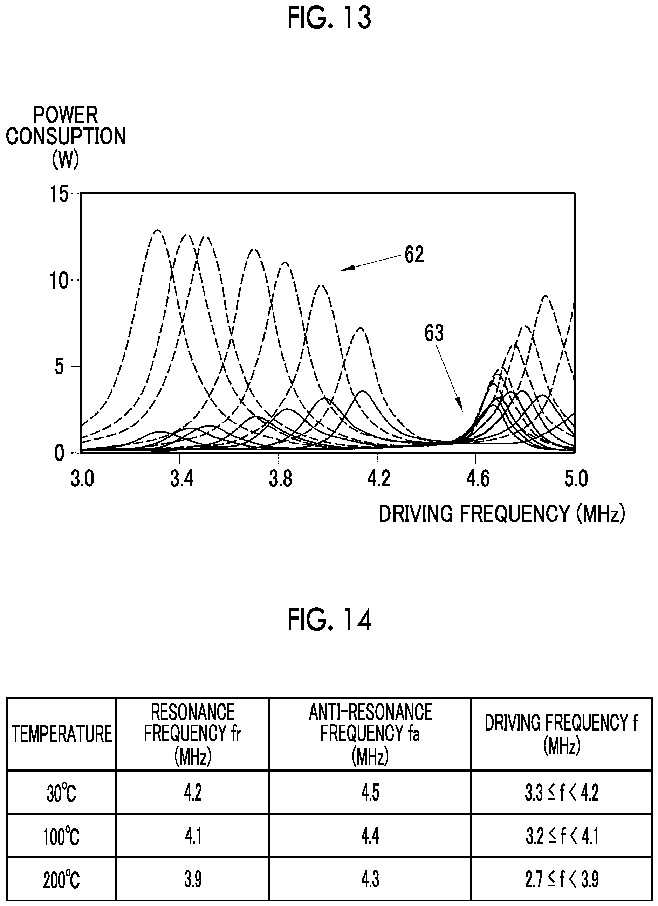

Fig. 13 shows a breakdown of probe power consumption accompanied by impedance adjustment. Power consumption contributing to self-heating of the probe corresponds to a difference between total power consumption and power consumption contributing to generation of ultrasound oscillation. A power consumption curve group 62 shown by a dotted line is total power consumption in a value of each capacitor C, and a power consumption curve group 63 shown by a solid line is power consumption related to ultrasound oscillation in a value of each capacitor C. Therefore, it is desirable to select the optimal driving frequency f in consideration of a ratio of self-heating to ultrasound oscillation after securing the total power consumption of the probe and to perform impedance adjustment. Accordingly, the temperature rising time of the piezoelectric element 39 is shortened. As the value of the optimal driving frequency f is lower, the total power consumption of the probe 30 having the piezoelectric element 39 increases, and power consumption contributing to generation of ultrasound oscillation reduces.

The temperature dependence of the resonance frequency fr and the anti-resonance frequency fa of the probe 30 increases due to an ancillary environment of the piezoelectric element, that is, interference with the backing member 37, the acoustic matching layer 35, and the like. In this case, the driving frequency f is set in consideration of a target reaching temperature zone. Specifically, in a case where the resonance frequency fr of the probe 30 fluctuates in a range from the treatment start temperature to the target reaching temperature, the driving frequency f may be the driving frequency f satisfying an allowable range of the resonance frequency fr and the driving frequency f in each temperature zone between the treatment start temperature and the target reaching temperature of the probe 30.

The present invention drives the piezoelectric element 39 at a frequency lower than the resonance frequency fr of the piezoelectric element 39 in a state of being accommodated in the probe 30 with a state where ultrasound oscillation is also considerably generated as a condition, while mainly optimizing a driving condition for actively promoting self-heating of the piezoelectric element 39. An impedance matching circuit for this purpose is added to the surgical treatment apparatus 20. The actual driving frequency f does not need to be exact.

As shown in Fig. 14, at 30°C, the resonance frequency fr is 4.2 MHz, the anti-resonance frequency fa is 4.5 MHz, and a range of the driving frequency f is 3.3 MHz or more and less than 4.2 MHz. At 100°C, the resonance frequency fr is 4.1 MHz, the anti-resonance frequency fa is 4.4 MHz, and the range of the driving frequency f is 3.2 MHz or more and less than 4.1 MHz. At 200°C, the resonance frequency fr is 3.9 MHz, the anti-resonance frequency fa is 4.3 MHz, and the range of the driving frequency f is 2.7 MHz or more and less than 3.9 MHz. In a case where the common driving frequency f is selected in a range including each temperature zone of 30°C, 100°C, and 200°C, the range of the driving frequency f is 3.3 MHz or more and less than 3.9 MHz. A method of calculating the range of the driving frequency at each temperature will be described later.

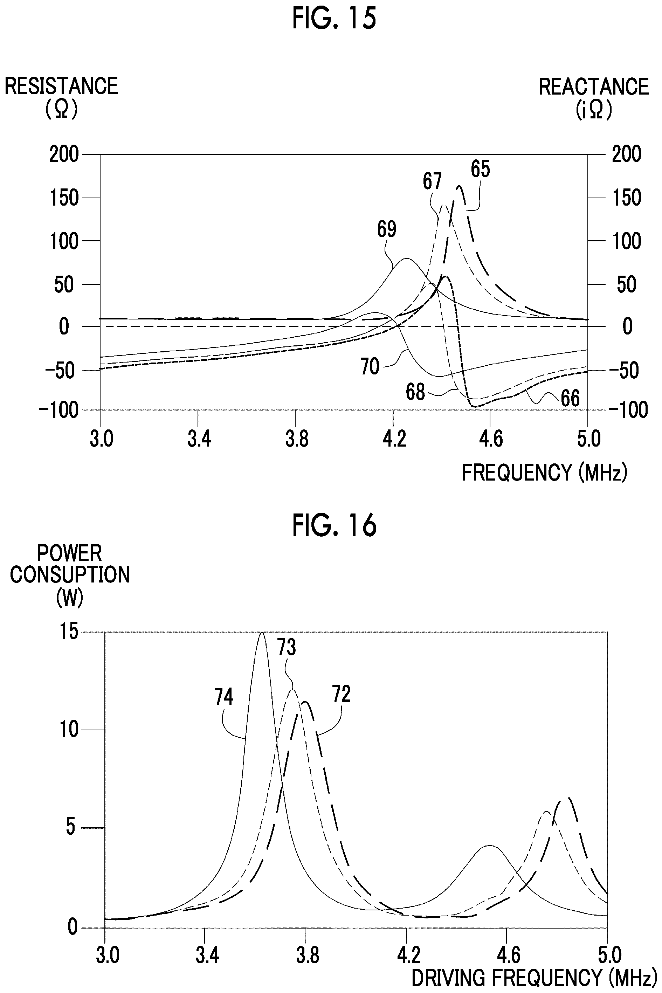

Fig. 15 shows temperature dependence of a probe impedance characteristic in a range of the treatment start temperature of 30°C and the maximum reaching temperature of 200°C. A 30°C curve 65 indicates a resistance in a case of 30°C, and a 30°C curve 66 indicates a reactance in a case of 30°C. A 100°C curve 67 indicates a resistance in a case of 100°C, and a 100°C curve 68 indicates a reactance in a case of 100°C. A 200°C curve 69 indicates a resistance in a case of 200°C, and a 200°C curve 70 indicates a reactance in a case of 200°C. In addition, the resonance frequency fr and the anti-resonance frequency fa in this case are as shown in Fig. 14. Each of the 30°C curve 66, the 100°C curve 68, and a 100°C curve 70 has a frequency at which the reactance component is 0 Ω, and the range of the optimal driving frequency f at each temperature is lower than a value of the frequency.

As shown in Fig. 16, the driving frequency f at which total power consumption is maximized is different for each temperature zone. As shown in a 30°C power consumption curve 72, the total power consumption is maximized near 3.8 MHz around 30°C. As shown in a 100°C power consumption curve 73, the total power consumption is maximized near 3.7 MHz around 100°C. In addition, as shown in a 200°C power consumption curve 74, the total power consumption is maximized near 3.6 MHz around 200°C. The probe 30 has a lower value of the optimal driving frequency as the reaching temperature of the piezoelectric element 39 is higher.

The amount of heat generated by the piezoelectric element 39 is controlled by adjusting a voltage or a current in a power supply circuit. In addition, in general, the driving frequency f during a temperature rise is fixed, but in a case where the temperature dependence of the resonance frequency fr of the probe 30 is large, a function of sequentially changing to the driving frequency f optimal for a probe temperature based on probe temperature data obtained by a temperature determination device (not shown) of the probe 30 may be provided.

In the present invention, a biological tissue is gripped by the probe 30 including the piezoelectric element 39, heat from self-heating of the piezoelectric element 39 is conducted to the biological tissue with the start of supply of predetermined power, and ultrasound oscillation generated in association with the self-heating is added, so that sealing of the biological tissue is effectively performed by a synergistic effect of heat, oscillation, and a grip pressure.

The driving frequency f of the probe 30 is a frequency lower than the resonance frequency fr of the probe 30 and there is the impedance matching circuit 44 in which a reactance component of impedance of the probe 30 is 0 Ω at the driving frequency f. The driving frequency f of the probe is provided in a range for efficiently raising the temperature, the range being determined by the resonance frequency fr of the probe and the anti-resonance frequency fa of the probe.

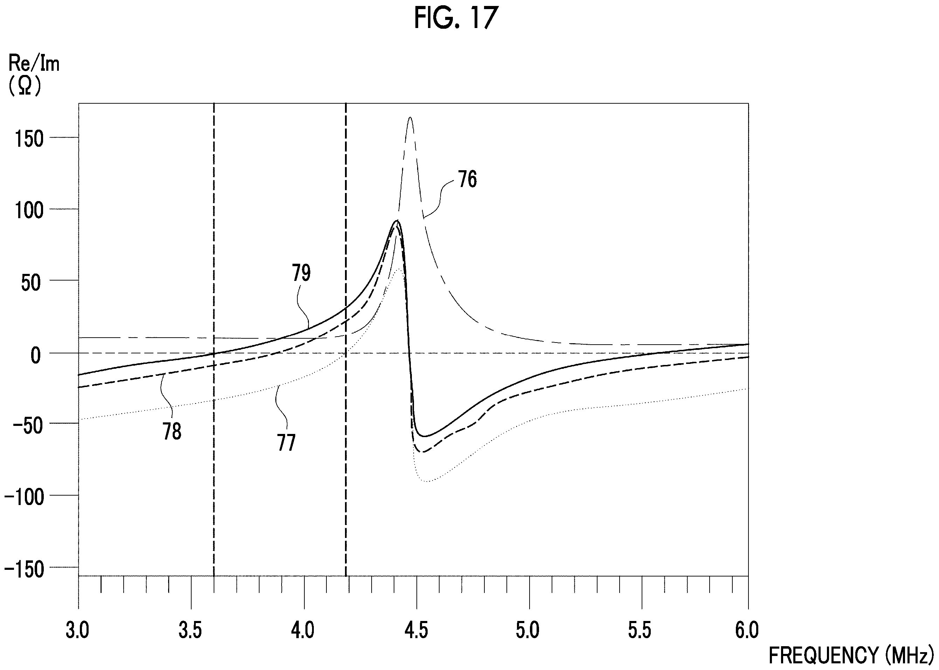

Fig. 17 is results of impedance measurement and impedance adjustment in a case where the piezoelectric element 39 accommodated in the probe 30 is a product having a thickness of 0.5 mm, which is C-213. In the impedance adjustment, the driving frequency f is shifted to the low range side. A curve 76 is a resistance curve of an impedance characteristic. A curve 77 is a reactance curve of the impedance characteristic, and the driving frequency f is 4.2 MHz. An adjustment curve 78 is a case where the driving frequency f is adjusted to 3.9 MHz, and an adjustment curve 79 is a case where the driving frequency f is adjusted to 3.6 MHz. A frequency at which the curve 77 has a frequency of 0 Ω on the low range side is the resonance frequency fr, and a frequency on a high range side is the anti-resonance frequency fa. A frequency at which reactance components of the adjustment curve 78 and the adjustment curve 79 are 0 Ω is the driving frequency f on the low range side and is approximately 4.5 MHz, that is, the anti-resonance frequency fa on the high range side.

The amount of heat generated by the piezoelectric element 39 in the probe 30 increases with the shift of the driving frequency f to the low range side, the amount of heat transmitted to a biological tissue, that is, a temperature rising speed of the biological tissue increases, and a treatment time until sealing is shortened. Probe power consumption depends on an applied voltage, and a desired temperature rising speed and a desired target reaching temperature are maintained by adjusting an alternating current voltage.

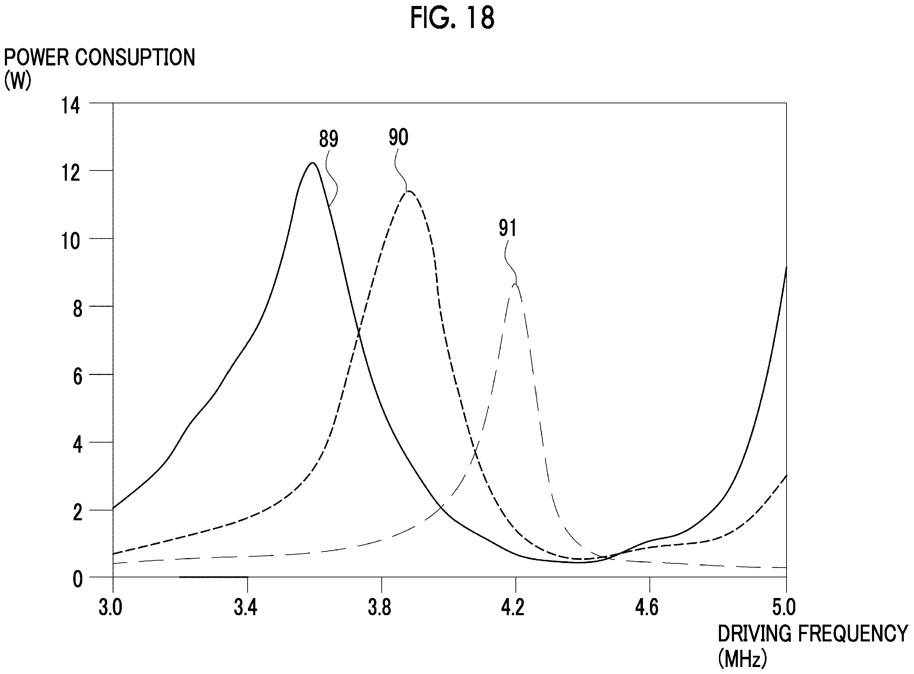

As shown in Fig. 18, in the present measurement frequency range, power consumption of the probe 30 increases as the adjusted driving frequency f is closer to the low range side. For probe power consumption in a case where a voltage of 10 V is applied, power consumption of a power consumption curve 90 in a case where the driving frequency f is adjusted to 3.9 MHz is larger than that of a power consumption curve 91 in a case where the driving frequency f is adjusted to 4.2 MHz, and power consumption of a power consumption curve 89 in a case where the driving frequency f is adjusted to 3.6 MHz is even larger. That is, with the adjustment, total power consumption of the probe 30 increases as the optimal driving frequency f takes a lower value.

As described above, regarding the range of the driving frequency f in impedance characteristic of the probe 30 alone, it is preferable that a relationship between the driving frequency f, the resonance frequency fr, and the anti-resonance frequency fa of the probe satisfies the following expression (6). The driving frequency f is a value lower than the resonance frequency fr. In a case where the driving frequency f is set to a frequency lower than the above range, ultrasound oscillation of the probe 30 is hardly obtained. A value used in calculating the range of the driving frequency f is the value shown in Fig. 14.

$$4fr - 3fa \leq f < fr \quad (6)$$

As a range of the driving frequency f of the probe 30 that is more desirable than that from expression (6), it is preferable to satisfy the following expression (7).

$$2.5fr - 1.5fa \leq f \leq 1.5fr - 0.5fa \quad (7)$$

As the most desirable driving frequency f of the probe 30, the following expression (8) centered in the range of expression (7) is preferably satisfied.

$$f = 2fr - fa \quad (8)$$

In the present embodiment, both the pair of grip pieces 31a and 31b constituting the probe 30 comprise the ultrasound oscillator 36. However, without being limited thereto, the ultrasound oscillator 36 may be provided at only one of the pair of grip pieces 31a and 31b. In this case, it is preferable that the ultrasound oscillator 36 is disposed parallel to the inner surface of one grip piece 31 and the inner surface of the other grip piece 31 facing the ultrasound oscillator 36 is formed of a material reflecting ultrasound oscillation caused by the ultrasound oscillator 36.

In the present embodiment, hardware structures of processing units, which perform various types of processing, such as the control mechanism 41, are various types of processors to be described below. The various types of processors include a central processing unit (CPU) that is a general-purpose processor which functions as various types of processing units by executing software (program), a graphical processing unit (GPU), a programmable logic device (PLD) that is a processor capable of changing a circuit configuration after manufacture, such as a field programmable gate array (FPGA), and a dedicated electric circuit that is a processor having a circuit configuration specifically designed to execute various types of processing.

One processing unit may be composed of one of the various types of processors or may be composed of a combination of two or more processors of the same type or different types (for example, a combination of a plurality of FPGAs, a combination of a CPU and an FPGA, or a combination of a CPU and a GPU). In addition, one processor may constitute a plurality of processing units. As an example in which one processor constitutes a plurality of processing units, first, there is a form in which one processor is configured by a combination of one or more CPUs and software and the processor functions as the plurality of processing units, as represented by a computer such as a client and a server. Second, there is a form in which a processor that realizes functions of the entire system including the plurality of processing units with one integrated circuit (IC) chip is used, as represented by a system on chip (SoC). As described above, the various types of processing units are composed of one or more of the various types of processors used as a hardware structure.

Further, the hardware structures of the various types of processors are, more specifically, an electric circuit (circuitry) in a form in which circuit elements such as semiconductor elements are combined. In addition, a hardware structure of a storage unit is a storage device such as a hard disc drive (HDD) and a solid state drive (SSD).

In addition, in the embodiment, the endoscope 12 to be combined with the ultrasound treatment tool 21 according to the embodiment of the present invention is not specified, and the endoscope 12 need only comprise the forceps channel into which the treatment tool is inserted, for example, a bronchoscope, an upper gastrointestinal endoscope, a lower gastrointestinal endoscope, or the like. It is preferable that the surgical treatment apparatus 20 having the impedance matching circuit 44 of the present embodiment is realized during manufacturing in a factory or the like.

Explanation of References

10: endoscope system
12: endoscope
12a: insertion part
12b: operating part
12c: bendable part
12d: distal end part
12e: angle knob
12f: forceps port
12g: forceps channel
12h: forceps outlet

14: light source device

15: processor device

16: display

17: user interface

20: surgical treatment apparatus

21: ultrasound treatment tool

21a: flexible tubular sheath

21b: operating part

22: ultrasound drive unit

30: probe

31: grip piece

31a: grip piece

31b: grip piece

32: opening and closing mechanism

33: support member

34: operation wire

35: acoustic matching layer

36: ultrasound oscillator

37: backing member

38a: electrode

38b: electrode

39: piezoelectric element

40: air gap layer

41: control mechanism

42: signal transmitter

43: amplifier

44: impedance matching circuit

47: frequency monitor

50: curve

51: curve

52: curve

53: power consumption curve

54: power consumption curve

55: power consumption curve

59: curve

60: curve

62: power consumption curve group

63: power consumption curve group

65: 30°C curve

66: 30°C curve

67: 100°C curve

68: 100°C curve

69: 200°C curve

70: 200°C curve

72: 30°C power consumption curve

73: 100°C power consumption curve

74: 200°C power consumption curve

76: curve

77: curve

78: adjustment curve

79: adjustment curve

89: power consumption curve

90: power consumption curve

91: power consumption curve

AC: power source

C: capacitor

C0: capacitor

C1: capacitor

Cs: capacitor
Dx: thickness direction
fa: anti-resonance frequency
f: driving frequency
fr: resonance frequency
IL: current
L: inductor
L0: inductor
Ls: inductor
R0: resistance
Rs: resistance
S: structure
Y: grip direction

**Claims**

1. A surgical treatment apparatus comprising:

   a probe that includes a piezoelectric element;
   a power supply circuit for driving the piezoelectric element;
   a power supply wiring line to the piezoelectric element;
   a flexible tubular sheath;
   a control mechanism of an amount of power supplied to the piezoelectric element; and
   an impedance matching circuit,
   wherein the probe has electrode layers for power supply on both surfaces of the piezoelectric element and an opening and closing mechanism for gripping a biological tissue,
   the flexible tubular sheath includes the power supply wiring line to the piezoelectric element and a part of the opening and closing mechanism of the probe, and
   the impedance matching circuit sets a driving frequency used in the power supply to the piezoelectric element to a frequency lower than a resonance frequency in an impedance characteristic of a treatment start temperature of the probe and a frequency at which a reactance component of impedance at the driving frequency is 0 Ω.

2. The surgical treatment apparatus according to claim 1,
   wherein in a case where the driving frequency of the probe is denoted by f, the resonance frequency is denoted by fr, an anti-resonance frequency is denoted by fa, a range of the driving frequency in the impedance characteristic of the treatment start temperature of the probe alone satisfies the following expression (1),

$$4\mathrm{fr} - 3\mathrm{fa} \leq f < \mathrm{fr} \quad (1).$$

3. The surgical treatment apparatus according to claim 1,
   wherein a range of the driving frequency in the impedance characteristic of the treatment start temperature of the probe alone satisfies the following expression (2),

$$2.5\mathrm{fr} - 1.5\mathrm{fa} \leq f \leq 1.5\mathrm{fr} - 0.5\mathrm{fa} \quad (2).$$

4. The surgical treatment apparatus according to claim 1,
   wherein a range of the driving frequency in the impedance characteristic of the treatment start temperature of the probe alone satisfies the following expression (3),

$$f = 2\mathrm{fr} - \mathrm{fa} \quad (3).$$

5. The surgical treatment apparatus according to claim 1,
   wherein the piezoelectric element consists of a rectangular planar shape and is polarized in a thickness direction.

6. The surgical treatment apparatus according to claim 1,

wherein one surface of the piezoelectric element is fixed to the probe with a backing member sandwiched therebetween, and
the other surface of the piezoelectric element is covered with at least one of or both an acoustic matching layer and an insulating layer.

7. The surgical treatment apparatus according to claim 1,
wherein the biological tissue is a soft tissue including a blood vessel.

8. The surgical treatment apparatus according to claim 1,
wherein the driving frequency is a frequency lower than the resonance frequency and is a frequency at which power consumption contributing to generation of ultrasound oscillation at the driving frequency is in a range of at least 25% or more of power consumption contributing to generation of ultrasound oscillation at the resonance frequency.

9. The surgical treatment apparatus according to any one of claims 1 to 8,
wherein in the probe, the resonance frequency in an impedance characteristic of a maximum reaching temperature of the piezoelectric element is equal to or lower than the resonance frequency in the impedance characteristic of the treatment start temperature, and a difference between the resonance frequency in the impedance characteristic of the maximum reaching temperature of the piezoelectric element and the resonance frequency in the impedance characteristic of the treatment start temperature is equal to or less than two times a difference between the resonance frequency and an anti-resonance frequency in the impedance characteristic of the treatment start temperature of the piezoelectric element.

# FIG. 1A

# FIG. 1B

# FIG. 2

# FIG. 3

(A)

OPENING AND
CLOSING OPERATION

(B)

# FIG. 4

(A)

OPENING AND
CLOSING OPERATION

(B)

# FIG. 5

## FIG. 6

EP 4 501 256 A1

## FIG. 7

## FIG. 8

# FIG. 9

# FIG. 10

# FIG. 11

# FIG. 12

# FIG. 13

# FIG. 14

| TEMPERATURE | RESONANCE FREQUENCY fr (MHz) | ANTI-RESONANCE FREQUENCY fa (MHz) | DRIVING FREQUENCY f (MHz) |
|---|---|---|---|
| 30°C | 4.2 | 4.5 | $3.3 \leq f < 4.2$ |
| 100°C | 4.1 | 4.4 | $3.2 \leq f < 4.1$ |
| 200°C | 3.9 | 4.3 | $2.7 \leq f < 3.9$ |

## FIG. 15

## FIG. 16

FIG. 17

EP 4 501 256 A1

FIG. 18

## INTERNATIONAL SEARCH REPORT

| | |
|---|---|
| International application No. | |
| | **PCT/JP2023/001918** |

### A. CLASSIFICATION OF SUBJECT MATTER

***A61B 17/32***(2006.01)i
FI: A61B17/32 510

According to International Patent Classification (IPC) or to both national classification and IPC

### B. FIELDS SEARCHED

Minimum documentation searched (classification system followed by classification symbols)

A61B17/32

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2017/191683 A1 (OLYMPUS CORP) 09 November 2017 (2017-11-09)<br>paragraphs [0014]-[0015], [0035], [0043]-[0055], fig. 2-3 | 1-9 |
| A | JP 2002-153480 A (TOSHIBA CORP) 28 May 2002 (2002-05-28)<br>paragraph [0017] | 1-9 |
| A | JP 2013-528071 A (KONINKLIJKE PHILIPS ELECTRONICS N.V) 08 July 2013<br>(2013-07-08)<br>paragraphs [0009]-[0011] | 1-9 |
| A | WO 2018/020553 A1 (OLYMPUS CORP) 01 February 2018 (2018-02-01)<br>paragraphs [0024], [0036]-[0037], [0040] | 1-9 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **06 March 2023** | **14 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/001918**

| Patent document cited in search report | | | Publication date (day/month/year) | Patent family member(s) | | | Publication date (day/month/year) |
|---|---|---|---|---|---|---|---|
| WO | 2017/191683 | A1 | 09 November 2017 | US 2019/0046226 A1 paragraphs [0023]-[0024], [0046], [0055]-[0068], fig. 2-3 | | | |
| JP | 2002-153480 | A | 28 May 2002 | (Family: none) | | | |
| JP | 2013-528071 | A | 08 July 2013 | WO 2011/148314 A1 p. 3, line 1 to p. 4, line 2 US 2013/0066240 A1 CN 102917756 A | | | |
| WO | 2018/020553 | A1 | 01 February 2018 | US 2019/0150975 A1 paragraphs [0028], [0040]-[0041], [0044] | | | |

Form PCT/ISA/210 (patent family annex) (January 2015)

**EP 4 501 256 A1**

**Patent documents cited in the description**

- JP 2013528071 A
- JP 2015043879 A
- WO 2017038722 A